Europäisches Patentamt

European Patent Office

Office européen des brevets

(19)

(11) Numéro de publication: **0 185 584**
**B1**

(12) **FASCICULE DE BREVET EUROPEEN**

(45) Date de publication du fascicule du brevet:
**14.09.88**

(51) Int. Cl.⁴: **A 61 K 33/24**

(21) Numéro de dépôt: **85402392.6**

(22) Date de dépôt: **03.12.85**

(54) Utilisation de l'hétéropolyanion (NaSb9W21O86)18- pour fabriquer une composition pharmaceutique destinée à être utilisée dans le traitement du sida et des syndromes analogues.

(30) Priorité: **03.12.84 US 678240**

(43) Date de publication de la demande:
**25.06.86 Bulletin 86/26**

(45) Mention de la délivrance du brevet:
**14.09.88 Bulletin 88/37**

(84) Etats contractants désignés:
**AT BE CH DE FR GB IT LI LU NL SE**

(56) Documents cités:
**FR - A - 2 245 374**
**FR - A - 2 334 366**

(73) Titulaire: **INSTITUT PASTEUR, 25-28, rue du Docteur Roux, F-75724 Paris Cédex 15 (FR)**
Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Chermann, Jean-Claude, 2 Clos d'Ergal, F-78310 Elancourt (FR)**
Inventeur: **Montagnier, Luc, 21 rue de Malabry, F-92350 Plessis-Robinson (FR)**
Inventeur: **Vilmer, Etienne, 80 rue de Lycée, F-92380 Sceaux (FR)**
Inventeur: **Dormont, Dominique, 119 Elysée II Route de la Jonchère, F-78120 La Celle-St-Cloud (FR)**
Inventeur: **Spire, Bruno, 25 rue des Jardins, F-92380 Garches (FR)**
Inventeur: **Barre-Sinoussi, Françoise, 104 Le Capricorne 50 rue d'Erevan, F-92130 Issy-les-Moulineaux (FR)**
Inventeur: **Rozenbaum, Willy, 11 passage de la Main d'Or, F-75011 Paris (FR)**

(74) Mandataire: **Harlé, Robert et al, c/o Cabinet Harlé & Phélip 21, rue de la Rochefoucauld, F-75009 Paris (FR)**

## Description

La présente invention concerne un médicament destiné au traitement du SIDA, syndrome immunodéficitaire acquis, et des syndromes analogues; elle concerne également une composition pharmaceutique contenant ce médicament.

Le principe actif de la nouvelle composition pharmaceutique selon la présente invention est connu; c'est l'hétéropolyanion de formule $(Na\ Sb_9\ W_{21}\ O_{86})^{18-}$, qui est présent dans la nouvelle composition sous forme de l'un de ses sels alcalins et/ou alcalino-terreux et/ou d'ammonium. L'étude de la structure de cet ion est décrite dans l'article de J. Fisher et coll., «J. Am. Chem. Soc.», 98 (10), 3050 (1976). L'utilisation de cet hétéropolyanion pour le traitement de certaines infections virales à l'origine de sarcomes et leucémies, de l'encéphalomyocardite ou de stomatites vésiculaires murines est décrite dans les demandes de brevets FR-A-2 245 374 et FR-A-2 334 366.

Les sels précités forment de nombreux hydrates. Le sel d'ammonium et de sodium de l'hétéropolyanion, ayant pour formule $[Na\ SB_9\ W_{21}\ O_{86}]\ (NH_4)\ _{17}Na,\ 14H_2O$ est généralement appelé HPA 23. Ce sel est un cryptate.

On peut rappeler que le HPA 23 est actif contre le développement de la scrapie chez la souris (Prion). A la suite de ces résultats, un essai clinique préliminaire sur une soixantaine de malades a été entrepris contre la maladie de Creutzfeldt-Jakob. Une stabilisation et une amélioration de certains patients ont été observées à des doses quotidiennes variant autour de 3 mg/kg.

On a maintenant trouvé que le HPA 23 et les autres sels de l'hétéropolyanion étaient utiles pour le traitement du SIDA; il n'existe aucun traitement connu de la lymphadénopathie qui se manifeste dans cette maladie, et l'administration d'un inhibiteur de la transcriptase inverse, enzyme nécessaire à la multiplication du rétrovirus à l'origine du SIDA, présente un intérêt non négligeable dans la mesure où cela limitera l'infestation virale in vivo.

On sait que le rétrovirus appelé LAV ou encore $HTLV_3$ est présent chez les malades atteints du SIDA; des variants et des mutants de ce virus ont aussi été récemment isolés.

La présente invention a d'abord pour objet l'utilisation d'un sel alcalin et/ou alcalino-terreux et/ou d'ammonium de l'hétéropolyanion $(NaSb_9W_{21}O_{86})^{18-}$, éventuellement sous une forme hydrate, pour fabriquer une composition médicamenteuse destinée au traitement du SIDA et des syndromes analogues chez les animaux à sang chaud, et, en particulier chez l'homme.

La présente invention concerne aussi un procédé de préparation d'une composition pharmaceutique destinée au traitement du syndrome immunodéficitaire acquis et des syndromes analogues caractérisé par le fait qu'on mélange au moins un sel alcalin et/ou alcalino-terreux et/ou d'ammonium de l'hétéropolyanion $(NaSb_9W_{21}O_{86})^{18-}$, éventuellement sous forme hydrate, avec un ou plusieurs diluants adjuvants compatibles, pharmaceutiquement acceptables.

La composition pharmaceutique selon l'invention peut être administrée par voie parentérale, orale, rectale, ou intralymphatique. La dose journalière par kilo de poids corporel sera de 0,1 à 100 mg. Parmi les voies d'administration parentérale, la voie intraveineuse est particulièrement adaptée.

Selon l'invention, une composition injectable ou administrable par voie intralymphatique, est obtenue par dissolution de la quantité convenable du sel de l'hétéropolyanion dans un solvant stérile pour injection, tel qu'une solution aqueuse isotonique de chlorure de sodium ou de mannitol, par exemple. La dissolution pourra être extemporanée, et la composition pharmaceutique se présentera sous forme d'un flacon contenant la dose unitaire de poudre stérile de sel et d'une ampoule contenant le solvant stérile pour injection; ou encore la solution injectable, prête à l'emploi, sera présentée en ampoules préparées dans les conditions habituelles. La composition pharmaceutique selon l'invention pourra aussi être mélangée à tout autre adjuvant compatible et pharmaceutiquement acceptable, notamment à un liquide de perfusion compatible, choisi parmi ceux habituellement utilisés tels qu'un osluté de glucose ou de chlorure de sodium par exemple, ou à un adjuvant tel que le mannitol ou le glucose par exemple.

La composition pharmaceutique selon l'invention pour administration par voie orale pourra se présenter sous l'une des formes habituelles, tels que comprimé, gélule, cahet, dragée; de préférence, cette forme comportera un enrobage protecteur (par exemple entérique).

La préparation des compositions pharmaceutiques selon l'invention est réalisée selon des méthodes classiques, connues du galéniste.

Dans ce qui suit on décrit les résultats du traitement par le HPA 23 de malades atteints du SIDA, ainsi que l'action du HPA 23 in vitro sur le LAV et sur un rétrovirus qui déclenche chez le singe, mais non chez l'homme, une lymphadénopathie; ce virus du singe a notamment été décrit dans Science 223 p. 1083–1086 (1984) et on a préconisé d'utiliser comme modèle animal du SIDA, l'infetion causée par ce rétrivorus chez le singe.

Des études cinétiques in vitro sur des cultures cellulaires infestées ont montré que les sels de l'anion de formule $(Na\ Sb_9\ W_{21}\ O_{86})^{18-}$, dont le HPA 23, étaient des inhibiteurs compétitifs de la transcriptase inverse du virus LAV comme à l'origine du SIDA simiesque. Ces sels ont été également trouvés actifs sur les enzymes de réplication d'autres rétrovirus tels que, par exemple, le virus de la leucémie de Moloney et de Rauscher, les virus murins, aviaires et humains (avec le $HTLV_1$).

Activité in vitro du HPA 23 sur le virus du SIDA simiesque et sur le virus LAV

a) Préparation de la transcriptase inverse du virus simiesque.

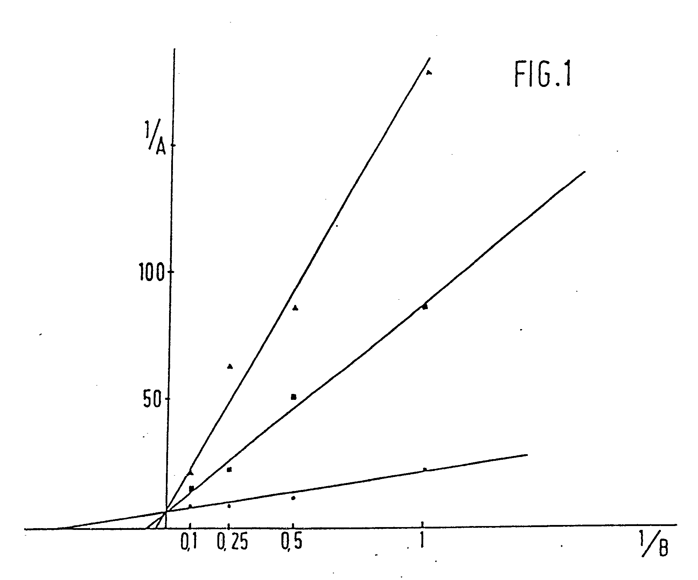

Les surnageants de cultures cellulaires infestées par le virus simiesque, provenant du laboratoire de Murray Gardner à l'Université de Californie (USA), ont été précipités par addition de polyéthylèneglycol (PEG) à 10% et le précipité a été purifié en gradient de saccharose de 20 à 60% (2,5 heures à 50 000 g). On a recherché la présence de l'enzyme transcriptase inverse sur chacune des fractions du gradient qui ont été isolées (voir la légende de la fig. 1). L'activité transcriptase inverse maximale a été trouvée pour la densité de 1,18.

b) Préparation de la transcriptase inverse du LAV

Le LAV, virus associé aux lymphadénopathies, a été purifié comme indiqué dans l'article de Science 220 868–871 (1983), c'est-à-dire qu'après clarification du surnageant des cultures cellulaires inféstées par le virus, on a ajouté du PEG 10% pour précipiter les virus et centrifugé le précipité pendant 1,5 heure à 55 000 g en gradient de saccharose de 10 à 60%. Le maximum d'activité de la transcriptase inverse a été trouvé pour la densité 1,16; cette fraction, considérée comme la fraction virale purifie, a été isolée.

c) Purification de la transcriptase inverse du LAV

On a ensuite isolé la transcriptase inverse en appliquant la méthode décrite dans Biochem. Biophys. Res. Comm. 121 126–133 (1984). Cette méthode consiste à laver 2 fois les cellules infectées par le LAV, de la fraction purifiée obtenue en b) par du tampon phosphate salin avant de les congeler à la température de l'azote liquide, en suspension dans un solvant aqueux contenant du tris (50 mM) (pH = 7,5), du KCl (500 mM), de l'EDTA (0,1 mM), ou acide éthylènediaminetétraacétique, du thiothreitol (1 mM) et du glycérol 10%. Après décongélation à 37 °C dans un solution à 0,01% di Triton® × 100, les homogénats cellulaires sont centrifugés à 100 000 g pendant une heure et le surnageant dialysé durant 6 heures contre KCl aqueux (100 mM). Le dialysat est chromatographié sur une colonne de phosphocellulose et on isole la transcriptase inverse et la DNA polymérase du virus en éluant avec un gradient de KCl aqueux (0,1 M à 1 M).

Dans le tableau I figurent les résultats des dosages de transcriptase inverse exprimés en picomoles de monophosphate de déoxyribonucléotide tritié incorporé dans l'ADN après 1 heure d'incubation à 37 °C.

Tableau I

| Virus | inhibiteur HPA 23 en µg/ml | picomoles incorporées | inhibition % | $DI_{50}$ µg/ml |
|---|---|---|---|---|
| Virus simiesque | 0 | 0,43 | 0 | |
| | 0,5 | 0,34 | 21 | |
| | 1 | 0,28 | 35 | |
| | 2,5 | 0,09 | 80 | 1,1 |
| | 5 | 0,024 | 94,5 | |
| | 10 | 0,0025 | 99,5 | |
| | 25 | 0 | 100 | |

| Virus | inhibiteur HPA 23 en µg/ml | picomoles incorporées | inhibition % | $DI_{50}$ µg/ml |
|---|---|---|---|---|
| Virus LAV | 0 | 0,13 | 0 | |
| | 10 | 0,127 | 2,4 | |
| | 25 | 0,013 | 18,6 | 18,6 |
| | 50 | 0,098 | 84,4 | |
| | 60 | 0 | 100 | |
| transcriptase inverse purifiée | 0 | 0,11 | 0 | |
| | 0,5 | 0,105 | 4,5 | |
| | 1 | 0,101 | 8,2 | |
| | 5 | 0,073 | 33,6 | |
| | 10 | 0,062 | 43,6 | 11 |
| | 20 | 0,042 | 61,8 | |
| | 30 | 0,032 | 70,2 | |
| | 60 | 0,007 | 94 | |
| | 100 | 0 | 100 | |

Le milieu d'incubation contenait Tris (50 mM) pH = 7,9; Mg Cl₂ (5 mM); KCl (20 mM); dithiothréitol (2 mM); Triton® × 100 (0,01%); poly A (0,05 OD/ml); oligo dT 12–18 (0,05 OD/ml); ³HTMP (200 pM-25 Ci/mM).

On constate que la transcriptase inverse du virus simiesque est fortement inhibée par le HPA 23, et que l'inhibition est fonction de la dose. La concentration de HPA 23 dans le milieu d'incubation inhibant la réaction à 50% ($DI_{50}$) est 1,1 µg/ml. Pour le LAV isolé selon b), la $DI_{50}$ est plus élevée: 30 µg/ml, tandis que lorsqu'on introduit la transcriptase inverse purifiée de LAV, isolée selon c), la $DI_{50}$ est de 11 µg/ml. Cette dernière valeur est inférieure à celle obtenue avec la transcriptase inverse non purifiée probablement parce qu'il y a un meilleur contact entre l'enzyme et le HPA 23.

Effet de la concentration du substrat (acide nucléique synthétique : matrice-amorceur) sur l'inhibition par le HPA 23, de la transcriptase inverse purifiée du LAV

Les résultats des diagrammes des Lineweaver-Burk des effets de la concentration variable de poly An oligo JT12–18 à deux concentrations différentes en HPA 23 sont résumés sur la fig. 1. Dans les conditions expérimentales, les paramètres enzymatiques cinétiques sont: KM = 2,18 nM de poly A oligo dT, Vmax = 0,02 pM de 3 HTMP incorporés par seconde. Cette figure illustre le mécanisme d'inhibition par compétitive du HPA 23, comme décrit préalablement avec l'inhibition de la transcriptase inverse du Virus de la Leucémie Murine (MLV) par le HPA 23. L'interaction du HPA 23 avec le complexe enzyme-acide nucléique (matrice-amorceur) devrait se situer au niveau de la liaison avec l'acide nucléique (matrice-amorceur).

Le mécanisme de l'inhibition de la transcriptase inverse est identique à celui montré dans d'autres modèles de rétrovirus. Par conséquent, l'inhibition par compétitive apparaît être le mécanisme général de l'action du HPA 23 sur la transcriptase inverse, même si les doses nécessaires sont différentes dans les modèles humain et animal. Le HPA 23 a également été découvert comme étant actif sur le système nerveux central et comme étant un agent stimulant de la fonction des cellules destructrices naturelles (NK) et des celles responsables de la cytotoxité dépendant d'anticorps (ADCC). Ceci suggère que l'activité en vivo de ce composé sur les infections pa le rétrovirus puisse être expliquée par les effets à la fois sur le système immunitaire et sur la réplication virale. Le SIDA simiesque peut être utilisé comme modèle pour expérimenter l'action des médicaments antiviraux malgré les différences dans les modèles clinique et immunologique du SIDA et du SIDA simiesque.

De nombreuses thérapies ont été proposées pour traiter les patients affectés par le SIDA, mais aucune preuve de thérapie réussie n'a été rapportée en dépit des effets limités sur les paramètres soit biologiques soit cliniques. Comme mentionné ci-dessus, le HPA 23 agit sur le système immunitaire aussi bien que, d'une manière spécifique, sur l'enzyme nécessaire pour la réplication virale (RT); en outre, des études sérologiques ont démontré qu'un pourcentage élevé d'anticorps anti LAV peut être détecté dans le complexe associé au SIDA (ARC) et chez les patients atteints de SIDA.

Légende de la fig. 1.

Représentation de Lineweaver-Burk de l'influence de la concentration en amorceur poly A oligo $T_{12-18}$ sur l'inhibition par le HPA 23 de la transcriptase inverse du LAV purifiée.

A: picomoles de $^3H$ TMP incorporées en 5 min à 37 °C.

B: concentration en acide nucléique (matrice amorceur) $(ODm^1)$.

●—●: Témoin – (pas d'inhibition)

■—■: la dose inhibitrice correspond à 65% d'inhibition par le HPA 23 (19 µg/ml).

▲—▲: la dose inhibitrice correspond à 80% d'inhibition par le HPA 23 (40 µg/ml).

Chaque point du diagramme est la moyenne de 3 expériences.

Inhibition de la transcriptase du virus HTLV-1 Par l'HPA-23

Introduction

Cette étude a pour but de déterminer un effet de l'HPA-23 sur la transcriptase inverse (RT) du virus HTLV-1.

Méthodes
Source de virus

Le virus HTLV-1 étant produit par des lignées continues, les présents inventeurs ont utilisé comme source de virus un mélange de surnageant de culture de lignées infectées par le HTLV-1 (CI0, MT2, C91). L'activité RT de ce pool viral est de 30 000 cpm/ml. Le virus est concentré 100 fois par ultracentrifugation sur un rotor IEC (1 h 30 à 38 000 tmp). Le culot viral est suspendu dans du tampon NTE (NaCl) 100 mM; Tris 10 mM pH 7,4; EDTA 1 mM et est conservé à –80 °C.

Source d'HPA-23

Les présents inventeurs ont utilisé de la poudre du tungsto-antimoniate telle que nous l'ont fournie MM. Hervé et Thézé (Faculté des Sciences de Paris). La poudre est dissoute dans de l'eau pyrolysée. Une solution mère à 50 mg/ml est effectuée.

Dosage de l'activité RT

Les présents inventeurs ont dosé l'activité RT de 10 µl de virus HTLV-1 concentré 100 fois en présence de concentrations variables d'HPA-23. L'activité RT est mesurée par l'incorporation de Thymidine tritiée dans une matrice synthétique; le polyr A en présence d'une source oligo dT. Le mélange réactionnel utilisé est celui nécessaire afin d'obtenir une activité enzymatique optimale. Il contient:

Tris pH 7,9 50 mM (Merck)
Chlorure de magnésium ($MgCl_2$) 5 mM (Prolabo)
Chlorure de potassium (KCl) 20 mM (Prolabo)
Dithiotreïtol (DTT) 1 mM (Sigma)
Polyr A 0,050 D/ml (Boehringer)
Oligo dT 12–18 0,05 OD/ml (PL Biochemicals)
Triton × 1000 0,1%
Thymidine triphosphate tritiée ($^3H$ TTP) $17,10^{-5}$ µM
Activité specifique 30 Ci/mmol (Amercham)
Les concentrations d'HPA-23 utilisées sont 0; 0,5; 1; 5; 10; 25; 50; 75; 100; et 1000 µg/ml.

Resultats

Ils sont indiqués sur le tableau Ia. Le pourcentage d'inhibition est représenté comme fonction de la concentration d'HPA-23. On en déduit la dose inhibitrice 50 (DI 50) qui est de 36 µg/ml.

Conclusion

L'HPA-23 inhibe la transcriptase inverse du virus HTLV-1. La DI 50 est du même ordre de grandeur (un peu supérieure) à celle déterminée pour le virus LAV. Elle est nettement supérieure à celle des rétrovirus murins (MuLV).

Tableau Ia
Inhibition de l'activité transcriptase inverse du virus HTLV-1 par l'HPA-23

| Echantillon | cpm | % d'inhibition |
|---|---|---|
| Témoin positif (virus MLV) | 797,375 | – |
| Témoin négatif | 1,700 | – |
| Témoin d'inhibition (MLV+HPA-23 à 25 µg/ml | 2,402 | 99,9 |
| HPA-23 = 0 | 30,272 | – |
| HPA-23 = 0,5 µg/ml | 31,695 | 0 |
| HPA-23 = 1 µg/ml | 30,687 | 0 |
| HPA-23 = 5 µg/ml | 31,520 | 0 |
| HPA-23 = 10 µg/ml | 27,070 | 11,2 |
| HPA-23 = 25 µg/ml | 24,092 | 21,6 |
| HPA-23 = 50 µg/ml | 12,638 | 61,7 |
| HPA-23 = 75 µg/ml | 7,975 | 78 |
| HPA-23 = 100 µg/ml | 6,028 | 84,8 |
| HPA-23 = 1000 µg/ml | 1,957 | 99,1 |

Application du HPA 23 au traitement du SIDA chez l'homme

Quatre malades dont les cellules sanguines contenaient des LAV ont reçu du HPA 23. L'état clinique des malades avant traitement est résumé dans le tableau II. Le premier malade a reçu le HPA 23 par voie intraveineuse, par une injection bolus en deux minutes environ, à des doses comprises entre 1 mg/kg et 3,3 mg/kg; deux traitements successifs respectivement de 25 et 53 jours ont été pratiqués, avec un intervalle d'environ deux mois.

On a constaté que le médicament était éliminé rapidement de l'organisme (demi-vie inférieure à 20 min); aussi les malades suivants ont reçu le HPA 23 en perfusion lente: 200 mg de sel en solution dans 250 ml de glucose isotonique, injecté en 3 heures. Ce traitement a été répété chaque jour pendant 15 jours.

Pour chacun de ces malades, on a étudié avant et après traitement, la quantité de virus apparue dans les cultures de leurs lymphocytes T. Les cultures ont été réalisées de façon classique, dans un milieu RPMI 1640 supplémenté avec 10% de sérum de veau fétal, du facteur de croissance TCGF, de l'interleukine 2, du sérum anti-alpha-interféron humain, du polybrène et des antibiotiques. On a mesuré la quantité de virus apparue dans les surnageants des cultures en déterminant l'activité transcriptase inverse 2 fois chaque semaine.

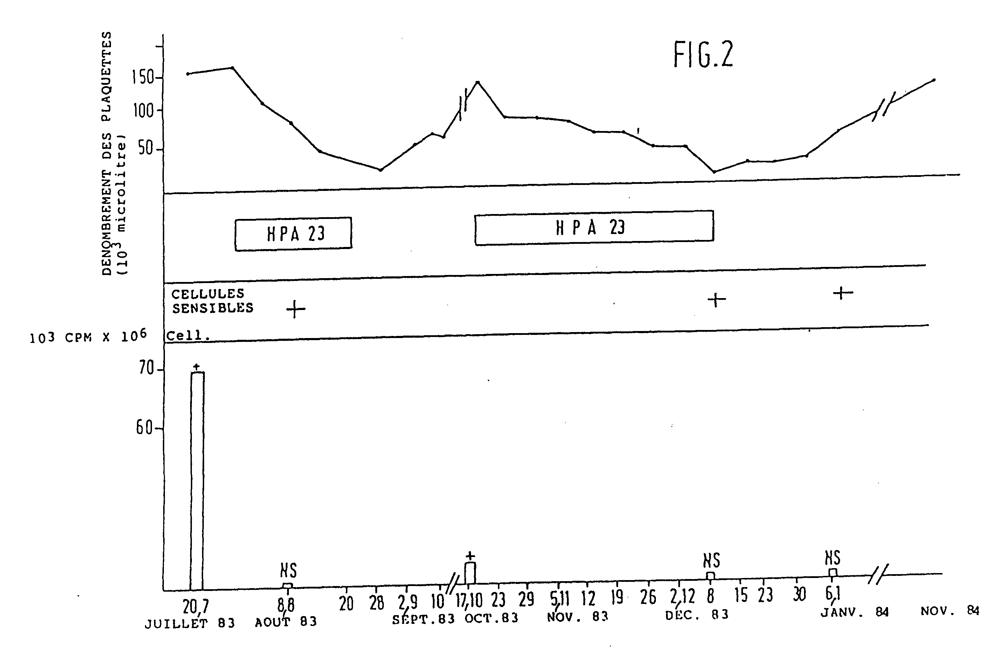

Pour le malade 1, le virus était absent des cultures cellulaires pendant chaque période de traitement mais est réapparu entre les deux traitements; après le second traitement, le virus LAV n'a pas été mis en évidence dans la culture de ses lymphocytes T, alors qu'on a pu montrer que cette culture pouvait être infectée par le virus isolé auparavant chez ce malade, ce qui prouvait la persistence chez lui de cellules sensibles au LAV. Les résultats sont résumés sur la fig. 2.

La fig. 2 résume la réplication du LAV testé par l'activité transcriptase inverse dans les surnageants de cultures de cellules T et le graphique du dénombrement des plaquettes pendant le traitement du patient 1 par le HPA 23. Ce patient a eu deux périodes de traitement: la première période étant celle du 27 juillet 1983 au 20 août 1983 et la deuxième période du 17 octobre 1983 au 8 décembre 1983. Les doses cumulatives étaient respectivement de 890 mg et 1980 mg. Dans chaque période les doses de HPA 23 ont été légèrement augmentées de 1 mg/kg à 3,3 mg/kg. Sur la fig. 2, «NS» veut dire «non significatif», autrement dit qu'il n'y a pas eu de virus détectable.

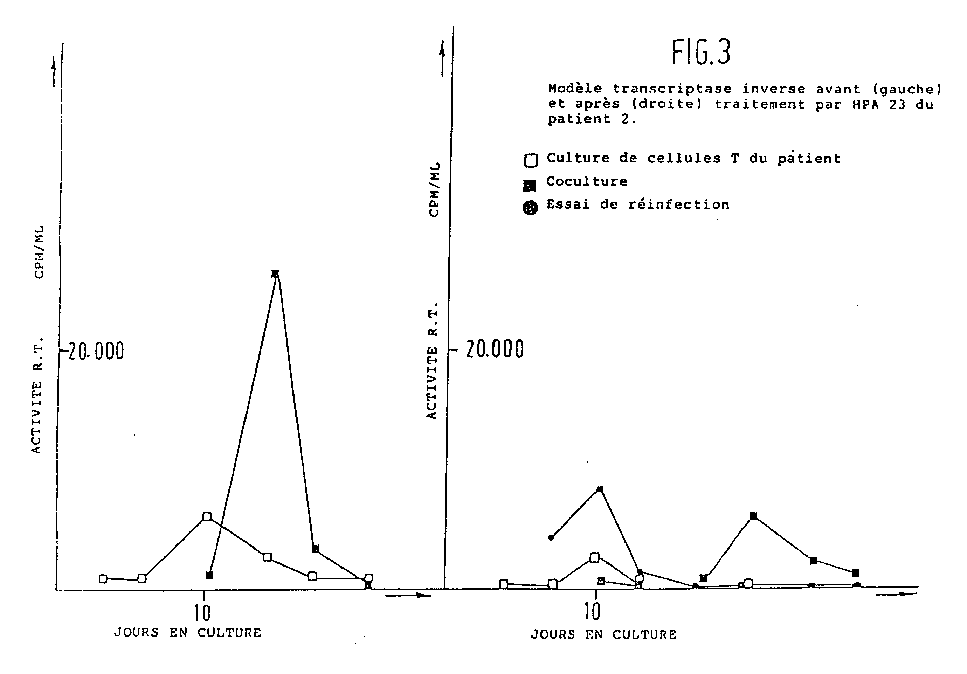

Pour les malades 2, 3, 4, chez lesquels on avait constaté la présence du LAV juste avant le début du traitement, après 2 semaines de traitement, le virus avait disparu des cultures cellulaires de leurs lymphocytes T, comme des cocultures avec les lymphocytes T provenant d'hommes sains. Toutefois, le virus est réapparu en faible quantité, 30 jours après la fin du traitement dans les cocultures résultant de prélèvements chez les malades 3 et 4, et chez les 4 malades, on a décelé quelques cellules sensibles aux virus et non infectées (fig. 3).

Lorsque le virus n'a pas été mis en évidence dans les surnageants de cultures, on a vérifié que les cellules pouvaient cependant être infectées par le virus; ceci traduit bien le fait que le HPA 23 inhibe la réplication virale, mais ne tue pas les cellules infectées par le virus et confirme les résultats obtenus in vitro; les doses qui bloquent la transcriptase inverse sont inférieures à celles inhibant les ADN-polymérases cellulaires et, par conséquent, ces doses ne sont pas cytotoxiques.

Par ailleurs, on a noté que le nombre total des lymphocytes $T_4+$ et le rapport $T_4/T_8$ n'a pas été sensiblement modifié chez les malades après l'arrêt du traitement. Par contre, le nombre des plaquettes a commencé à diminuer chez deux malades dès le 8ème jour du traitement et cela a continué jusqu'à son interruption. Pour chacun des adultes traités, le même nombre de plaquettes qu'avant le traitement a été retrouvé entre le 21ème et le 45ème jour après son arrêt. De faibles augmentations des transaminases hépatiques (deux fois la valeur normale) ont été notées chez les malades 2, 3 et 4 pendant le traitement; la situation s'est rétablie dans les 21 jours qui ont suivi son arrêt. Enfin on n'a pas observé la toxicité rénale.

Les observations ci-dessus montrent que la composition pharmaceutique contenant le HPA 23 comme principe actif est utile au traitement du SIDA.

Tableau II

| Ma-lade | Sexe | Age | Nature du risque | Date de diagnostic | Manifestation clinique | nombre abs. de cell $T_4+$ | Date de détection du LAV | Présence d'anticorps anti-LAV* | Début traitement | Etat au SO/11/84 |
|---|---|---|---|---|---|---|---|---|---|---|
| 1 | M | 13 | hémophile | Mai 83 | toxoplasmose | 9 | Mai 83 | + | 26/7/83 | vivant |
| 2 | F | 30 | Haïti | Mai 84 | PCP* | 32 | Mai 84 | + | 7/7/84 | vivant |
| 3 | M | 30 | homo-sexuel | Avril 84 | sarcome de Kaposi PCP* | 141 | Mai 84 | + | 11/6/84 | vivant |
| 4 | M | 30 | Haïti | Avril 84 | lympha-dénopathie | 170 | Mai 84 | + | 17/6/84 | vivant |

\* PCP: Pneumocystis carinii pneumoniae
(×)+ signifie ELISA et RIPA (35 Sméthionine)

Aussi les compositions pharmaceutiques comportant l'hétéropolyanion $(Na\,Sb_9\,W_{21}\,O_{86})^{18-}$ associé à d'autres principes actifs utiles au traitement du SIDA et aux excipients usuels, sont un objet de l'invention. Ces compositions peuvent se présenter sous forme de dose unitaire unique, contenant l'ensemble des constituants en mélange, ou encore fractionnée, les composants devant être administrés simultanément ou séparément.

Dans ce qui suit, on décrit des exemples de préparation de compositions pharmaceutiques selon l'invention.

Le principe actif $[Na\,Sb_9\,W_{21}\,O_{86}](NH_4)_{17}Na$, $14H_2O$ est préparé selon le procédé connu, décrit dans le brevet des Etats-Unis d'Amérique n° 4 547 369.

### Exemple A

On prépare un soluté injectable par voie intra-veineuse, prêt à l'emploi, dosé à 2,5%, en répartissant par ampoule:

| | |
|---|---|
| HPA 23 | 50 mg |
| chlorure de sodium | 9 mg |
| Eau pour préparations injectables qsp. | 2 cm³ |

Ce soluté est stocké à +40 °C jusqu'à son utilisation.

### Exemple B

On prépare des flacons de poudre stérile destinée à une administration par voie intra-veineuse et des ampoules de solvant, en effectuant la répartition suivante:

Flacon de poudre stérile:

| | |
|---|---|
| HPA 23 | 50 mg |

Ampoule de solvant:

| | |
|---|---|
| Soluté isotonique de chlorure de sodium | 2 cm³ |

### Exemple C

On prépare des flacons de poudre stérile destinée à une administration par voie intra-veineuse et des ampoules de solvant, en effectuant la répartition suivante:

Flacon de poudre stérile:

| | |
|---|---|
| HPA 23 | 50 mg |
| Mannitol | 100 mg |

Ampoule de solvant:

| | |
|---|---|
| Eau pour préparations injectables | 2 cm³ |

### Revendications

1. Utilisation d'un sel alcalin et/ou alcalino-terreux et/ou d'ammonium de l'hétéropolyanion $(NaSb_9\,W_{21}\,O_{86})^{18-}$ éventuellement sous une forme hydrate, pour fabriquer une composition médicamenteuse destinée au traitement du syndrome immunodéficitaire acquis (SIDA) et des syndromes analogues.

2. Utilisation selon la revendication 1, caractérisée par le fait que le sel est un sel d'ammonium et de sodium défini par la structure:

$$[NaSb_9W_{21}O_{86}](NH_4)_{17}\,Na\,14\,H_2O.$$

3. Utilisation selon l'une des revendications 1 et 2, en vue de la préparation d'une composition pharmaceutique administrable par voie parentérale, orale, rectale ou intralymphatique.

4. Utilisation selon la revendication 3, en vue de la préparation d'une composition pharmaceutique administrable par voie parentérale et se présentent notamment sous forme d'une dose unitaire.

5. Procédé de préparation d'une composition pharmaceutique destinée au traitement du syndrome immunodéficitaire acquis et des syndromes analogues, caractérisé par le fait qu'on mélange au moins un sel alcalin et/ou alcalino-terreux et/ou d'ammonium de l'hétéropolyanion $(NaSb_9W_{21}O_{86})^{18-}$, éventuellement sous une forme hydrate, avec un ou plusieurs diluants ou adjuvants compatibles pharmaceutiquement acceptables.

6. Procédé selon la revendication 5, caractérisé par le fait qu'on utilise le sel d'ammonium et de sodium défini par la structure $[NaSb_9W_{21}O_{86}]$ $(NH_4)_{17}\,Na$, $14H_2O$.

7. Procédé selon l'une des revendications 5 et 6, caractérisé par le fait qu'on prépare une composition administrable par voie parentérale, orale, rectale ou intralymphatique.

### Patentansprüche

1. Verwendung eines Alkali- und/oder Erdalkali- und/oder Ammonium-Salzes des Heteropolyanions $(NaSb_9W_{21}O_{86})^{18-}$, gegebenenfalls in Form eines Hydrats, zur Herstellung einer pharmazeutischen Zusammensetzung zur Behandlung von Aids und analoger Syndrome.

2. Verwendung nach Anspruch 1, dadurch gekennzeichnet, dass das Salz ein Ammonium- und Natriumsalz, das durch die Struktur $[NaSb_9W_{21}O_{86}](NH_4)_{17}Na \cdot 14H_2O$ definiert ist, ist.

3. Verwendung nach einem der Ansprüche 1 und 2 zum Zwecke der Herstellung einer pharmazeutischen Zusammensetzung für die parenterale, orale, rektale oder intralymphatische Verabreichung.

4. Verwendung nach Anspruch 3 zum Zwecke der Herstellung einer pharmazeutischen Zusammensetzung zur parenteralen Verabreichung, insbesondere in Form einer Einheitsdosis.

5. Verfahren zur Herstellung einer pharmazeutischen Zusammensetzung, die zur Behandlung von Aids und analoger Syndrome bestimmt ist, dadurch gekennzeichnet, dass man wenigstens ein Alkali- und/oder Erdalkali- und/oder Ammonium-Salz des Heteropolyanions $(NaSb_9W_{21}O_{86})^{18-}$, gegebenenfalls in Form eines Hydrats, mit einem oder mehreren pharmazeutisch akzeptablen Verdünnungsmitteln oder Hilfsstoffen mischt.

6. Verfahren nach Anspruch 5, dadurch gekennzeichnet, dass man das Ammonium- und das Natriumsalz verwendet, das durch die Struktur $[NaSb_9W_{21}O_{86}](NH_4)_{17}Na \cdot 14H_2O$ definiert ist.

7. Verfahren nach einem der Ansprüche 5 und 6, dadurch gekennzeichnet, dass man eine Zusammensetzung für die parenterale, orale, rektale oder intralymphatische Verabreichung herstellt.

## Claims

1. Use of an alkali metal salt and/or alkaline earth metal salt and/or ammonium salt of the heteropolyanion $(NaSb_9W_{21}O_{86})^{18-}$, optionally in a hydrated form, for manufacturing a medicinal composition intended for the treatment of acquired immune deficiency syndrome (AIDS) and similar syndromes.

2. Use according to claim 1, characterized in that the salt is an ammonium and sodium salt defined by the structure: $[NaSb_9W_{21}O_{86}](NH_4)_{17}Na \cdot 14H_2O$.

3. Use according to one of claims 1 and 2, for the purpose of preparing a pharmaceutical composition which is administrable parenterally, orally, rectally or intralymphatically.

4. Use according to claim 3, for the purpose of preparing a pharmaceutical composition which is administrable parenterally and takes the form, in particular, of a unit dose.

5. Process for preparing a pharmaceutical composition intended for the treatment of acquired immune deficiency syndrome and similar syndromes, characterized in that at least one alkali metal salt and/or alkaline earth metal salt and/or ammonium salt of the heteropolyanion $(NaSb_9W_{21}O_{86})^{18-}$, optionally in a hydrated form, is mixed with one or more pharmaceutically acceptable and compatible adjuvants or diluents.

6. Process according to claim 5, characterized in that the ammonium and sodium salt defined by the structure $[NaSb_9W_{21}O_{86}](NH_4)_{17}Na \cdot 14H_2O$ is used.

7. Process according to one of claims 5 and 6, characterized in that a composition which is administrable parenterally, orally, rectally or intralymphatically is prepared.

FIG.1

FIG.2

# FIG.3

Modèle transcriptase inverse avant (gauche)
et après (droite) traitement par HPA 23 du
patient 2.

☐ Culture de cellules T du patient
■ Coculture
● Essai de réinfection

CPM/ML

ACTIVITE R.T.

−20.000

−20.000

10

10

JOURS EN CULTURE

JOURS EN CULTURE

0185584